(19) Europäisches Patentamt
European Patent Office
Office européen des brevets

(11) Veröffentlichungsnummer: **0 023 033**
**A1**

# (12) EUROPÄISCHE PATENTANMELDUNG

(21) Anmeldenummer: **80104172.4**

(22) Anmeldetag: **17.07.80**

(51) Int. Cl.³: **C 07 D 239/30**

(30) Priorität: **21.07.79 DE 2929594**

(43) Veröffentlichungstag der Anmeldung:
**28.01.81 Patentblatt 81/4**

(84) Benannte Vertragsstaaten:
**BE CH DE FR GB IT LI NL**

(71) Anmelder: **HOECHST AKTIENGESELLSCHAFT**
**Zentrale Patentabteilung Postfach 80 03 20**
**D-6230 Frankfurt/Main 80(DE)**

(72) Erfinder: **Günther, Dieter, Dr.**
**Nachtigallenweg 1A**
**D-6233 Kelkheim (Taunus)(DE)**

(72) Erfinder: **Bosse, Dieter, Dr.**
**Berliner Strasse 12**
**D-6238 Hofheim am Taunus(DE)**

## (54) 2.4.6-Trihalogen-pyrimidin-5-carbonsäurehalogenide und Verfahren zu ihrer Herstellung.

(57) 2.4.6-Trihalogen-pyrimidin-5-carbonsäurehalogenide werden hergestellt durch Umsetzung von 2.4.6-Trihalogen-pyrimidin- 5-aldehyden mit Chlor und/oder wenigstens einem Chlorierungsmittel (Sulfurylchorid, etc.) gegebenenfalls in Gegenwart eines Radikalstarters bei Temperaturen von etwa 20 bis 150 °C in einem inerten organischen Lösungsmittel und ggf. Umhalogenierung der dabei gebildeten 2.4.6-Trihalogen-pyrimidin- 5-carbonsäurechloride mit üblichen Fluorierungs- oder Bromierungsmitteln. Die Produkte sind Zwischenprodukte insbesondere auf dem Farbstoffsektor.

EP 0 023 033 A1

0023033

HOECHST AKTIENGESELLSCHAFT HOE 79/F 201 Dr.ME/a

# 2.4.6-Trihalogen-pyrimidin-5-carbonsäurehalogenide und Verfahren zu ihrer Herstellung

Pyrimidinderivate sind wertvolle Zwischen- und Endprodukte auf verschiedenen Sachgebieten, z.B. auf dem Gebiet der Farbstoffe, der Pflanzenschutzmittel und der Pharmazeutika.

Ein interessantes Pyrimidinderivat ist u.a. auch das 2.4.6-Trichlor-pyrimidin-5-carbonsäurechlorid. Diese Verbindung ist in den beiden DE-OSen 2 113 298 und 2 208 972 unter einer Vielzahl möglicher Reaktivkomponenten zur Herstellung von Reaktivfarbstoffen aufgezählt, doch ist dort über ihre Herstellung nichts angegeben. Auch die übrige einschlägige Literatur - siehe z.B. die zwei auf ein Verfahren zur Herstellung von Halogen-pyrimidinen aus Nitrilen und Isocyanid-dichloriden gerichteten DE-OSen 1 670 854 und 1 770 774 - läßt nicht erkennen, wie man zu dem 2.4.6-Trichlor-pyrimidin-5-carbonsäurechlorid oder allgemein zu 2.4.6-Trihalogen-pyrimidin-5-carbonsäurehalogeniden gelangen kann.
Es bestand daher die Aufgabe, ein möglichst einfaches und wirtschaftliches Verfahren zur Herstellung des 2.4.6-Trichlor-pyrimidin-5-carbonsäurechlorids sowie auch anderer 2.4.6-Trihalogen-pyrimidin-5-carbonsäurehalogenide zu finden und diese Verbindungen damit der Technik zugänglich zu machen

Diese Aufgabe konnte erfindungsgemäß gelöst werden.

Erfindungsgegenstand sind daher zunächst 2.4.6-Trihalogen-pyrimidin-5-carbonsäurehalogenide der allgemeinen Formel I:

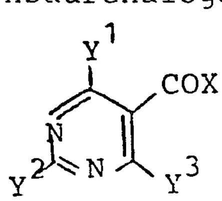

(I)

worin X, $Y^1$, $Y^2$ und $Y^3$ unabhängig voneinander

= F, Cl; Br.

BAD ORIGINAL

Unter diese Formel fallende Verbindungen sind z.B.

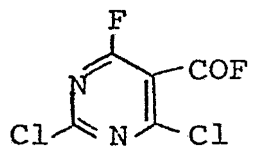 , 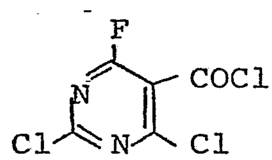 ,

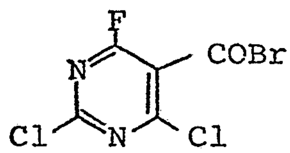 , 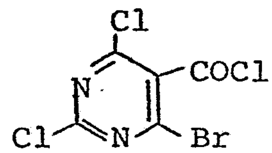 , etc.

Bevorzugte 2.4.6-Trihalogen-pyrimidin-5-carbonsäurehalogenide sind diejenigen unter die Formel I fallenden Verbindungen, in welchen $X = Y^1 = Y^2 = Y^3$; d. i. also:

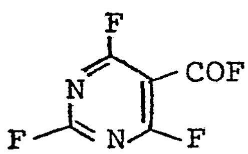 , 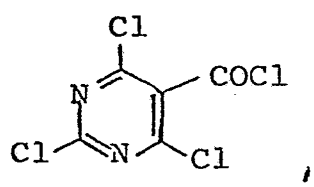 , 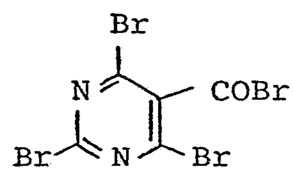

Besonders bevorzugte Verbindung ist die Verbindung der Formel I mit $X = Y^1 = Y^2 = Y^3 = Cl$, d.i. also: 2.4.6-Trichlor-pyrimidin-5-carbonsäurechlorid.

Die Verbindungen der Formel I werden erfindungsgemäß dadurch hergestellt, daß man 2.4.6-Trihalogen-pyrimidin-5-aldehyde der allgemeinen Formel II

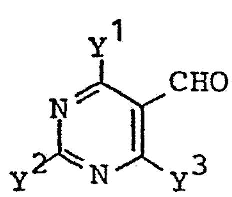 (II)

worin $Y^1$, $Y^2$ und $Y^3$ die gleiche Bedeutung wie in Formel I besitzen, mit Chlor und/oder einem Chlorierungsmittel ggf. in Gegenwart eines Radikalstarters bei Temperaturen zwischen etwa 20 und 150°C, vorzugsweise zwischen etwa 50 und 100°C, in einem inerten organischen Lösungsmittel umsetzt

und das dabei gebildete 2.4.6-Trihalogen-pyrimidin-5-carbonsäurechlorid anschließend ggf. mit üblichen Fluorierungs- oder Bromierungsmitteln umhalogeniert.

Es ist zwar bekannt, Benzaldehyd und einige speziell substituierte Benzaldehyde wie z.B. o-Chlorbenzaldehyd mit Hilfe von Chlor oder von Chlorierungsmitteln wie z.B. Sulfurylchlorid, Alkylhypochloriten, N-Chlorsuccinimid etc. in die entsprechenden Säurechloride zu überführen [s. z.B. H.T. Clarke und E.R. Taylor, Org. Synth. Coll. Vol. I, 155 (1947); H.C. Brown, Ind. Eng. Chem. 36, 788 (1944); S. Goldschmidt, R. Endres und R. Dirsch, Ber. 58, 576 (1925); Ginsburg, J. Amer. Chem. Soc. 73, 702 (1951); etc.]; im allgemeinen bleibt jedoch diese Reaktion entweder völlig aus oder führt lediglich zu kernchlorierten Benzaldehyd-Derivaten. Bei der Verwendung von Alkylhypochloriten als Chlorierungsmittel sind darüberhinaus die entsprechenden Carbonsäurechloride nur in wenigen Fällen isolierbar. Diese Umsetzung führt durch Reaktion der Säurechloride mit den intermediär gebildeten Alkoholen meist gleich zu Estern bzw. Carbonsäuren.

Zur Herstellung von heterocyclischen Carbonsäurechloriden aus den entsprechenden Aldehyden wurde diese Reaktion bisher noch nicht angewandt.

Formelmäßig läßt sich die Reaktion mit Chlor und den gängigsten Chlorierungsmitteln wie folgt wiedergeben (wobei von dem Ausgangs-Aldehyd der aromatische bzw. heterocyclische Rest weggelassen ist):

$$-CHO \quad + \quad Cl_2 \quad \longrightarrow \quad -COCl \quad + \quad HCl$$

$$-CHO \quad + \quad SO_2Cl_2 \quad \longrightarrow \quad -COCl \quad + \quad HCl + SO_2$$

-CHO +

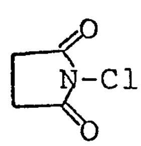

$\longrightarrow$ -COCl +

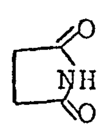

Als Ausgangsverbindungen der Formel II für das erfindungsgemäße Verfahren seien beispielsweise genannt:

2.4.6-Trichlor-pyrimidin-5-aldehyd, 2.4.6-Tribrom-pyrimidin-5-aldehyd, 2.4.6-Trifluor-pyrimidin-5-aldehyd, 4-Chlor-2.6-difluor-pyrimidin-5-aldehyd, 4-Chlor-2.6-dibrom-pyrimidin-5-aldehyd, 2-Chlor-4.6-difluor-pyrimidin-5-aldehyd, 2-Chlor-4.6-dibrom-pyrimidin-5-aldehyd, 2.4-Dichlor-6-fluor-pyrimidin-5-aldehyd, 4.6-Dichlor-2-fluor-pyrimidin-5-aldehyd, 2.4-Dichlor-6-brom-pyrimidin-5-aldehyd, 2.4-Dichlor-2-brom-pyrimidin-5-aldehyd, u.s.w.

Bevorzugte Ausgangsverbindungen sind 2.4.6-Trifluor-,
2.4.6-Trichlor- und
2.4.6-Tribrom-pyrimidin-5-aldehyd;
besonders bevorzugte Ausgangsverbindung ist 2.4.6-Trichlor-pyrimidin-5-aldehyd.

Herstellung der Ausgangsverbindungen:
2.4.6-Trichlor-pyrimidin-5-aldehyd kann z.B. gemäß der DE-OS 2 310 334 aus Barbitursäure, Phosphoroxichlorid und Dimethylformamid dargestellt werden. Andere 2.4.6-Trihalogen-pyrimidin-5-aldehyde können aus diesem Pyrimidinaldehyd nach bekannten Verfahren durch Umhalogenierung erhalten werden. Die Umhalogenierung kann z.B. erfolgen mit AgF /Wempen und Fox, J. Med. Chem. 6, 688 (1963); Horwitz und Thomson, J. Org. Chem. 26, 3392 (1961)/, KF (Horwitz und Thomson, a.a.O.), K-Fluorosulfinat (DE-OS 28 19 787, DE-OS 2 819 837), $PBr_3$ /Caton, Hurst, McOmie u. Hunt, J. Chem. Soc. (C) 1967, 1204/ etc.

Die Überführung der Ausgangs-aldehyde der Formel II in die entsprechenden Säurechloride geschieht mit Chlor und/oder einem Chlorierungsmittel; bevorzugte Chlorierungsmittel sind im vorliegenden Fall Sulfurylchlorid und N-Chlorsuccinimid. Es kann sowohl Chlor oder eines der genannten Chlorierungsmittel allein oder auch 2 oder mehrere Chlorierungsagenzien gemischt verwendet werden.

Für die Umsetzung ist es zweckmäßig, die Reaktionspartner in annähernd stöchiometrischem Verhältnis einzusetzen; das Chlorierungsagens kann jedoch auch im Überschuß vorliegen.

Als Radikalstarter dienen die zur Einleitung von Radikalreaktionen bekannten Verbindungen wie z.B. organische Peroxide (Benzoylperoxid etc.), organische Azoverbindungen (Azo-bis-Isobutylnitril etc.).

Der verwendete Radikalstarter wird zweckmäßig in katalytischen Mengen von etwa 0,1 bis 10 Gew.-%, bezogen auf den Ausgangs-aldehyd II, verwandt.

Die Reaktionstemperaturen liegen vorteilhaft im Bereich zwischen etwa 20 und 150°C, vorzugsweise von etwa 50 bis 100°C.

Als inerte organische Lösungsmittel kommen praktisch alle organischen Lösungsmittel in Frage, in welchen sich zumindest die Ausgangsverbindung löst und welche weder mit den Ausgangs- noch mit den End-Verbindungen noch mit den Chlorierungsagentien und den Radikalstartern in irgendeiner Weise reagieren. Bevorzugte inerte organische Lösungsmittel sind chlorierte aliphatische Kohlenwasserstoffe mit 1 bis 4 C-Atomen wie z.B. Chloroform, Tetrachlorkohlenstoff, Hexachlorethan etc.

Das Verhältnis von Lösungsmitteln zur Summe der Reaktanden kann in einem relativ weiten Bereich variiert werden; allgemein wird ein Gewichtsverhältnis von etwa 15 : 1 bis etwa 3 : 1 von Lösungsmittel zur Summe von Ausgangsaldehyd II und Chlorierungsagens bevorzugt.

Die Reaktionszeit beträgt je nach Reaktionstemperatur im Durchschnitt zwischen etwa 3 und 15 Stunden, vorzugsweise zwischen etwa 3 und 10 Stunden.

Bei der erfindungsgemäßen Umsetzung von 2.4.6-Trihalogen-pyrimidin-5-aldehyden mit Chlor und/oder einem der genannten Chlorierungsmittel entstehen zunächst nur die entsprechenden Säurechloride. Diese können - falls gewünscht - mittels üblicher Fluorierungs- oder Bromierungsmittel (Fluorierungsmittel: wasserfreie Flußsäure, Alkalimetallfluoride - vorzugsweise NaF, KF und CsF -, Ammoniumfluoride, $SbF_3$, $SF_4$, K-Fluorosulfinat etc.; Bromierungsmittel: $PBr_3$ etc.) auf an sich übliche Weise zu den entsprechenden Säurefluoriden bzw. -Bromiden umhalogeniert werden. Die Umhalogenierung kann - falls die Substituenten $Y^1$, $Y^2$ und/oder $Y^3$ nicht das gewünschte Halogen sind - auch noch weitergeführt werden bis zum Ersatz auch dieser Substituenten durch das gewünschte Halogen.

Für die Umhalogenierung kann das Halogenierungsmittel oft - wie z.B. bei Verwendung von $PBr_3$ als Umhalogenierungsmittel - auch als Lösungsmittel dienen.

Die Reaktionstemperatur für die Umhalogenierungen liegt zweckmäßig im allgemeinen zwischen etwa 20 und 250°C, vorzugsweise zwischen etwa 50 und 130°C.

Die erfindungsgemäßen 2.4.6-Trihalogen-pyrimidin-5-säurehalogenide der vorstehend angegebenen allgemeinen Formel I sind wertvolle Zwischenprodukte insbesondere auf dem Farbstoffsektor. So erhält man z.B. durch Umsetzung der Verbindungen der Formel I mit aminogruppenhaltigen Farbstoffen nach dem in den DE-OSen 2 113 298 und 2 208 972 angegebenen Reaktionsschema faserreaktive Farbstoffe.

Die folgenden Beispiele sollen der weiteren Erläuterung der Erfindung dienen.

Beispiel 1:

105,85 g (0.5 Mol) 2.4.6-Trichlor-pyrimidin-5-aldehyd werden in 500 ml trockenem Tetrachlorkohlenstoff gelöst und nach Zusatz von 100 mg Azobisisobutyronitril unter Rühren auf 60°C erhitzt. Anschließend tropft man langsam 67,48 g (0.5 Mol) Sufurylchlorid zu. Die Lösung erwärmt sich dabei auf Siedetemperatur und lebhafte Chlorwasserstoff und Schwefeldioxid-Entwicklung setzt ein. Nach 4-stündigem Erhitzen unter Rückfluß setzt man weitere 100 mg Azo-bis-isobutyronitril und 6.75 g (0.05 Mol) Sulfurylchlorid zu und erhitzt nochmals 4 Stunden auf Rückflußtemperatur.Die entstandene hellgelbe Lösung wird i. V. eingeengt. Der gelbe, teilweise kristalline Rückstand wird im Vakuum fraktioniert destilliert.

Ausbeute: 111.98 g 2.4.6-Trichlorpyrimidin-5-carbonsäurechlorid (91 Gew.-% d. Th.)
Kp.: 73 - 76° bei 1.63 mb
Fp.: 44 - 45°C.

Beispiel 2:

105,85 g (0,5 Mol) 2.4.6-Trichlor-pyrimidin-5-aldehyd und 87,73 g (0,65 Mol) Sulfurylchlorid in 500 ml Tetrachlorkohlenstoff werden wie unter Beispiel 1 beschrieben umgesetzt, jedoch wird die Reaktionszeit auf 12 Stunden verlängert.

Ausbeute: 117.52 g 2.4.6-Trichlor-pyrimidin-5-carbonsäurechlorid (96 Gew.-% d. Th.)
farblose Kristalle
Fp.: 44 - 46°C

Beispiel 3:

21,15 g (0,1 Mol) 2.4.6-Trichlor-pyrimidin-5-aldehyd werden in 100 ml trockenem Tetrachlorkohlenstoff suspendiert. Nach 10-minütigen Durchleiten von Stickstoff wird unter Rühren auf 70°C erwärmt, 100 mg Benzoylperoxid zugesetzt und sofort 13,5 g (0,1 Mol) Sulfurylchlorid zugetropft. Dabei schäumt die Reaktionsmischung stark auf. Nach 3-stündigen Rühren bei Rückflußtemperatur (78°C) läßt man abkühlen, dekantiert von geringen Mengen ausgefallenen Festkörper und entfernt das Lösungsmittel im Vakuum. Man erhält 16,72 g einer hellgelben, semikristallinen Masse. Vakuumdestillation liefert

13.01 g 2.4.6-Trichlor-pyrimidin-5-carbonsäurechlorid,
farblose Kristalle (53 Gew.-%)
Kp.: 73 - 76°C bei 1.6 mb
Fp.: 43 - 45°C

Beispiel 4:

4,92 g (20 mmol) des gemäß Beispiel 1 dargestellten 2.4.6-Trichlor-pyrimidin-5-carbonsäurechlorids werden mit 75 ml Phosphortribromid 5 Tage bei 110 - 120°C gerührt. Man entfernt das überschüßige Phosphortribromid i. Vakuum und sublimiert den Rückstand bei 120 - 130°C/0.15 mm.

Ausbeute: 6.41 g 2.4.6-Tribrom-pyrimidin-5-carbonsäurebromid
Fp.: 127 - 128°C.

Beispiel 5:

2,4,6-Trichlor-pyrimidin-5-carbonsäurebromid

21.15 g (0.1 Mol) 2,4,6-Trichlor-pyrimidin-5-aldehyd, 19.58 g (0.1 Mol) N-Bromsuccinimid und 100 mg 2,2'-Azobis(2-methylpropionitril) als Kettenstarter werden in 200 ml trockenem Tetrachlorkohlenstoff suspendiert und unter Rühren 3 Stunden auf Siedetemperatur erhitzt. Nach Abkühlen wird das feste Succinimid abfiltriert und das Lösungsmittel i.V. entfernt. Es verbleiben 34.1 g rohes Säurebromid, das durch Vakuumdestillation gereinigt wurde:

Ausbeute: 23.5 g (81 % d.Th.) 2,4,6-Trichlorpyrimidin-5-carbonsäurebromid

Fp.: 54 - 55 °C
Kp.: 84 - 90 °C bei 0.35 - 0.4 mm

IR: $\nu_{C=O}$ = 1795 $cm^{-1}$

Beispiel 6:

4,6-Dichlor-pyrimidin-5-carbonsäurechlorid

Eine Suspension von 17.7 g (0.1 Mol) 4,6-Dichlor-pyrimidin-5-aldehyd wird mit 14.85 g (0.11 Mol) Sulfurylchlorid und 100 mg 2,2'-Azobis(2-methylpropionitril) als Kettenstarter versetzt. Anschließend wird unter Rühren auf Siedetemperatur erhitzt. Nach 3,5 Stunden ist die lebhafte Chlorwasserstoff- und Schwefeldioxid-Entwicklung beendet. Die klare, hellgelbe Lösung wird i.V. vom Lösungsmittel befreit; es verbleiben 22.5 g rohes Säurechlorid, das durch Vakuumdestillation gereinigt wird:

Ausbeute: 19.05 g (90.1 % d. Th.)
4,6-Dichlor-pyrimidin-5-carbonsäurechlorid

Kp: 60 - 62 °C bei 0.4 mm

NMR ($CDCl_3$): = 8.9 ppm (1H, Singulett)

IR: $V_{C=O}$ = 1800 $cm^{-1}$

MS: Molekulion m/e = 210 (3 Cl)

Patentansprüche:

1. 2.4.6-Trihalogen-pyrimidin-5-carbonsäurehalogenide der allgemeinen Formel I

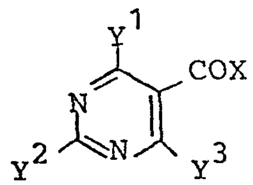

(I)

worin X, $Y^1$, $Y^2$ und $Y^3$ unabhängig voneinander = F, Cl, Br.

2. Verbindungen nach Anspruch 1, dadurch gekennzeichnet, daß in Formel I X = $Y^1$ = $Y^2$ = $Y^3$.

3. Verbindung der Formel I gemäß Anspruch 1 mit X = $Y^1$ = $Y^2$ = $Y^3$ = Cl.

4. Verfahren zur Herstellung der 2.4.6-Trihalogen-pyridin-5-carbonsäurehalogenide der allgemeinen Formel I gemäß Anspruch 1,
dadurch gekennzeichnet, daß man 2.4.6-Trihalogen-pyrimidin-5-aldehyde der Formel II

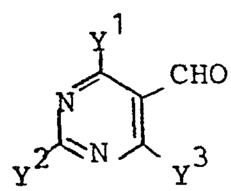

(II)

worin $Y^1$, $Y^2$ und $Y^3$ die gleiche Bedeutung wie in Formel I besitzen,

mit Chlor und/oder einem Chlorierungsmittel, gegebenenfalls in Gegenwart eines Radikalstarters, bei Temperaturen von etwa 20 bis 150°C, vorzugsweise von etwa 50 bis 100°C, in einem inerten organischen Lösungsmittel umsetzt und das dabei gebildete 2.4.6-Trihalogen-5-carbonsäurechlorid anschließend ggf. mit üblichen Fluorierungs- oder Bromierungsmitteln umhalogeniert.

5. Verfahren nach Anspruch 4, dadurch gekennzeichnet, daß man als Ausgangsverbindungen 2.4.6-Trihalogen-pyrimidin-5-aldehyde der Formel II mit $Y^1 = Y^2 = Y^3$ verwendet.

6. Verfahren nach Ansprüchen 4 bis 5, dadurch gekennzeichnet, daß man als Ausgangsverbindung den 2.4.6-Trihalogen-pyrimidin-aldehyd der Formel II mit $Y^1 = Y^2 = Y^3 = Cl$ verwendet.

7. Verfahren nach Ansprüchen 4 bis 6, dadurch gekennzeichnet, daß man die Umsetzung mit Chlor und/oder Sulfurylchlorid und/oder N-Chlorsuccinimid durchführt.

8. Verfahren nach Ansprüchen 4 bis 7, dadurch gekennzeichnet, daß man als inerte organische Lösungsmittel chlorierte aliphatische Kohlenwasserstoffe verwendet.

Europäisches Patentamt

# EUROPÄISCHER RECHERCHENBERICHT

0023033

Nummer der Anmeldung

EP 80104172.4

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| | DE - A - 1 770 049 (FARBENFABRIKEN BAYER)<br>+ Gesamt +<br>-- | 1,4,7,8 | C 07 D 239/30 |
| | DE - B - 2 064 096 (BAYER AG)<br>+ Patentanspruch +<br>-- | 1 | |
| | DE - A1 - 2 451 630 (BAYER AG)<br>+ Patentansprüche +<br>---- | 1 | |

RECHERCHIERTE SACHGEBIETE (Int. Cl.³)

C 07 D 239/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 26-09-1980 | LUX |